# EUROPÄISCHE PATENTSCHRIFT

(11) **EP 2 488 136 B1**
(45) Veröffentlichungstag und Bekanntmachung des Hinweises auf die Patenterteilung: **11.12.2013**
(21) Anmeldenummer: 10763321.6
(22) Anmeldetag: 05.10.2010
(51) Int. Cl.: A61F 13/15, A61F 13/66

(54) **FIXIER- UND TRAGEVORRICHTUNG FÜR EINE WEGWERFBARE ABSORBIERENDE INKONTINENZVORLAGE**
FASTENING AND CARRYING DEVICE FOR A DISPOSABLE ABSORBENT INCONTINENCE PAD
DISPOSITIF DE FIXATION ET DE SUPPORT POUR UNE PROTECTION POUR INCONTINENCE ABSORBANTE JETABLE

(30) Priorität: 15.10.2009 DE 102009049463
(43) Veröffentlichungstag der Anmeldung: 22.08.2012
(73) Patentinhaber: Paul Hartmann Aktiengesellschaft, 89522 Heidenheim (DE)
(72) Erfinder: PAZ, Rui, Miguel, 89518 Heidenheim (DE); KESSELMEIER, Rüdiger, 89542 Herbrechtingen (DE); GAUSE, Enno, 89522 Heidenheim (DE)
(74) Vertreter: Dreiss
(86) Internationale Anmeldenummer: PCT/EP2010/006066
(87) Internationale Veröffentlichungsnummer: WO 2011/044995

(56) Entgegenhaltungen:
- US-A- 791 354
- US-A- 1 475 895
- US-A- 2 017 499
- US-A- 4 022 212
- US-A1- 2004 186 456
- US-A1- 2004 267 225
- US-A1- 2006 247 599

## Beschreibung

Die Erfindung betrifft eine Fixier- und Tragevorrichtung für eine wegwerfbare absorbierende Inkontinenzvorlage, mit einem mittels Gürtelverschlusselementen auf sich selbst lösbar schließbaren und so eine in Hüftumfangsrichtung durchgehende Hüftöffnung bildenden Hüftgürtel, an den die Inkontinenzvorlage lösbar fixierbar ist, so dass sie im Schrittbereich des Benutzers getragen und nach Gebrauch wieder vom Hüftgürtel entfernt und verworfen werden kann, wobei der Hüftgürtel einen vorderen Bauchbereich, einen hinteren Rückenbereich und einen linken und einen rechten Seitenbereich umfasst, wobei der Hüftgürtel ausgehend von dem Rückenbereich und dem Bauchbereich je einen sich in einer Längsrichtung in Richtung auf den Schrittbereich des Benutzers erstreckenden Latzabschnitt aufweist, der an seiner körperzugewandten Seite vorzugsweise mechanisch wirkende Verschlusselemente aufweist, die mit komplementären vorzugsweise mechanisch wirkenden Verschlusselementen auf der körperabgewandten Seite der Inkontinenzvorlage lösbar haftend zusammenwirken.

US 2006/0247599 A1 zeigt eine solche Fixier- und Tragevorrichtung für eine wegwerfbare Inkontinenzeinlage, sofern man deren bekleidungsstückartige äußere Hüllschicht mit dem Hüftgürtel gemäß Diktion der vorliegenden Anmeldung assoziiert. Sinn und Zweck dieser bekleidungsstückartigen äußeren Hüllschicht ist es jedoch, die wegwerfbare absorbierende Einlage vollständig zu überdecken und einen sehr geringen Reibungskoeffizienten zwischen der Außenseite der absorbierende Einlage und der Innenseite der Hüllschicht zu verwirklichen. Dieser Reibungskoeffizient soll wenigstens 15 % geringer sein als der Reibungskoeffizient zwischen der Außenseite der Hüllschicht und einem angrenzenden Material, wie z.B. Bettlaken. Auf diese Weise werden in geringerem Umfang Verschiebungskräfte auf die absorbierende Einlage übertragen. Dies funktioniert aber natürlich nur dann, wenn durch die ausladende Gestaltung der bekleidungsstückartigen äußeren Hüllschicht die absorbierende Einlage derart von der Umgebung verhüllt und abgeschirmt ist, dass sie keinen Kontakt an ein angrenzendes Material, wie z.B. an einen Bettlaken, haben kann.

Eine andere Fixier- und Tragevorrichtung ist beispielsweise bekannt aus WO 2004/069122 A1. Bei dieser bekannten Fixier- und Tragevorrichtung wird der Hüftgürtel im Bauchbereich auf sich selbst geschlossen. In Seitenbereichen links und rechts zwischen Bauchbereich und Rückenbereich erstreckt sich der Hüftgürtel nach unten und bildet dort jeweils einen latzartigen Ansatz, an den eine verhältnismäßig breit ausladende absorbierende Windeleinheit festgelegt werden kann, wobei auf der körperzugewandten Seite der absorbierenden Windeleinheit beidseits vorn und hinten Verschlusselemente hierfür vorgesehen sind, festgelegt werden.

Auch EP 0 700 278 B1 zeigt eine sogenannte Gürtelwindel mit einem auf sich selbst schließbaren verhältnismäßig schmalen Hüftgürtel, an dessen körperabgewandter Seite eine nach Art einer Windel anmutende absorbierende Einheit festlegbar ist.

Der vorliegenden Erfindung liegt die Aufgabe zugrunde, eine Fixier- und Tragevorrichtung der eingangs genannten Art zu schaffen, die auch in Verbindung mit verhältnismäßig klein ausladenden Inkontinenzvorlagen verwendbar ist und dennoch im an den Benutzer angelegten Zustand eine höschenartig anmutende Konfiguration bildet und einfach handhabbar sein soll.

Diese Aufgabe wird durch eine Fixier- und Tragevorrichtung mit den Merkmalen des Anspruchs 1 gelöst..

Durch den in Richtung auf den Schrittbereich des Benutzers erstreckten Latzabschnitt bildet der Hüftgürtel zugleich die grundsätzliche Konfiguration eines Höschens, welches zur Vollendung der Höschenform nur noch durch einen Schrittbereich ergänzt zu werden braucht. Dieser Schrittbereich wird bei der erfindungsgemäßen Fixier- und Tragevorrichtung durch die absorbierende Inkontinenzvorlage gebildet, wobei der betreffende hintere und vordere Latzabschnitt mit der körperabgewandten Seite der Inkontinenzvorlage lösbar haftend zusammenwirkt, und zwar unter Verwendung von vorzugsweise mechanisch oder klebend wirkenden Verschlusselementen.

Zur Verbesserung der Passform und für eine einfache Handhabung erweist es sich als vorteilhaft, wenn der betreffende Latzabschnitt sich in Richtung auf den Schrittbereich verjüngt.

Weiter erweist es sich als vorteilhaft, wenn die Verschlusselemente des Latzabschnitts an einem schrittzugewandten Ende des Latzabschnitts vorgesehen sind. Darüber hinaus können am hinteren und/oder vorderen Latzabschnitt ein oder mehrere weitere Verschlusselemente vorgesehen sein, die zwischen dem schrittzugewandten Ende und dem Hüftgürtel positioniert sind und die der zusätzlichen Fixierung der Inkontinenzvorlage dienen.

Weiter erweist es sich als vorteilhaft, wenn die vorzugsweise mechanisch wirkenden Verschlusselemente des Latzabschnitts, insbesondere am schrittzugewandten Ende des Latzabschnitts, auf einem streifenförmigen und quer zur Längsrichtung erstreckten Materialabschnitt vorgesehen sind, der an den Latzabschnitt angefügt ist. Auf diese Weise kann der Latzabschnitt über seine gesamte flächenhafte Erstreckung in Querrichtung in einer bestimmungsgemäßen zum Benutzer symmetrischen Weise an der Außenseite der Inkontinenzvorlage lösbar festgelegt werden. Der Latzabschnitt kann so weitgehend faltenfrei und wie erwähnt zu den Beinen und zum Schrittbereich bzw. zu einer auf den Benutzer abstellenden Längsmittelachse korrekt ausgebreitet und fixiert werden.

Die Außenseite der Inkontinenzvorlage ist dabei so auszubilden, dass sie mit den Verschlusselementen des Latzabschnittes korrespondiert, das heißt, bei einem mechanischen Verschlusselement in Form von Haken wird die Außenseite der Inkontinenzvorlage vorzugsweise durch eine schlaufenbildende Komponente, vorzugsweise durch ein Vlies, insbesondere die Vlieslage eines Vlies-Folien-Laminates, gebildet, während bei einem klebenden Verschlusselement die Außenseite der Inkontinenzvorlage vorzugsweise durch eine Folienlage gebildet wird, auf der die klebenden Verschlusselemente anhaften können.

Nach einem weiteren Erfindungsgedanken erweist es sich als vorteilhaft, wenn die Verschlusselemente des Latzabschnitts, insbesondere am schrittzugewandten Ende, vor der Ingebrauchnahme auf die körperzugewandte Seite des Latzabschnitts eingeschlagen und in dieser Konfiguration leicht lösbar an der körperzugewandten Seite des Latzabschnitts haftend sind. Auf diese Weise sind die an dem Latzabschnitt vorgesehenen, insbesondere mechanisch wirkenden Verschlusselemente während des Anlegens des Hüftgürtels, insbesondere bei pflegebedürftigen Personen, so lange deaktiviert, wie sie nicht zum Festlegen der Inkontinenzvorlage benötigt werden. Sie behindern dabei nicht das Anlegen des Hüftgürtels, indem sie am Hüftgürtel oder an anderen Komponenten wie Bettlaken, Patientenunterlagen oder dergleichen, festhaken. In der Praxis kann es sich nämlich als vorteilhaft erweisen, wenn der vordere und/oder hintere Latzabschnitt auf sich selbst und/oder auf den Hüftgürtel gefaltet ist und die Faltung erst nach Anlegen des Hüftgürtels und der Inkontinenzvorlage geöffnet wird, um die Vorlage zu fixieren.

Im Hinblick auf die praktische Handhabbarkeit erweist es sich auch als vorteilhaft, dass der Hüftgürtel nur in einem Bereich öffenbar und schließbar und die Gürtelverschlusselemente in diesem Bereich an freien Endabschnitten des Hüftgürtels vorgesehen sind. Der Hüftgürtel ist also langgestreckt und hat zwei Endabschnitte, die mittels der Gürtelverschlusselemente aufeinander schließbar sind, um den Hüftgürtel um den Körperumfang des Benutzers herum anlegen zu können.

In Weiterbildung dieses Erfindungsgedankens erweist es sich als vorteilhaft, wenn im anderen Seitenbereich, in dem der Gürtel also nicht öffenbar und schließbar ist, Sekundärverschlusselemente vorgesehen sind, mittels derer eine Umfangslänge des Hüftgürtels einstellbar und so zumindest der vordere Latzabschnitt symmetrisch zum Schritt des Benutzers positionierbar ist. Vorzugsweise sind die Sekundärverschlusselemente so ausgebildet, dass sie im angelegten Zustand der Fixier- und Tragevorrichtung in der Ansicht optisch und/oder haptisch nicht von den Gürtelverschlusselementen unterscheidbar sind. Es kann sich jedoch auch als vorteilhaft erweisen, wenn die Sekundärverschlusselemente von den Gürtelverschlusselementen optisch und/oder haptisch unterscheidbar sind, um auf die unterschiedliche Funktion der Verschlusselemente hinzuweisen. Dies kann beispielsweise durch unterschiedliche Farben, Formen, Textur oder auf andere Weise erfolgen. Die Gürtelverschlusselemente und die Sekundärverschlusselemente sind beidseits des Bauchabschnitts und/oder in Seitenbereichen des Hüftgürtels vorgesehen. - Beim Anlegen des Hüftgürtels führt die Pflegeperson den geöffneten langgestreckten Hüftgürtel bei einem liegenden Patienten unter dessen Körper auf Hüft- oder Rückenhöhe hindurch. Sodann wird der Gürtel zur Bildung der geschlossenen Hüftöffnung mittels der Gürtelverschlusselemente auf sich selbst geschlossen. In diesem Zustand ist der Gürtel zwar angelegt, jedoch noch nicht durch Aktivierung der Sekundärverschlusselemente in seine optimale Passform gebracht. Beispielsweise wird in diesem Zustand der bauchseitige Latzabschnitt durch den Zug in Hüftumfangsrichtung, der durch das Schließen der Gürtelverschlusselemente ausgeübt wird, zu der betreffenden Seite hin gezogen. Dem kann nun durch Einstellen der optimalen Hüftumfangslänge und einer geeigneten Spannung in Hüftumfangsrichtung begegnet werden, indem die Sekundärverschlusselemente entsprechend optimal eingestellt werden. Mittels dieser Sekundärverschlusselemente werden also - wie bereits ausgeführt - nicht zwei offene freie Enden aufeinander geschlossen, sondern es wird die Umfangslänge des Hüftgürtels gegebenenfalls verstellt.

Die Sekundärverschlusselemente können in verschiedener Weise verwirklicht sein. Nach einer bevorzugten Ausführungsform der Erfindung wird vorgeschlagen, dass eine Komponente der Sekundärverschlusselemente auf einem an den Hüftgürtel angefügten Materialabschnitt vorgesehen ist und die andere Komponente an einer Außenseite des Hüftgürtels selbst vorgesehen ist. Solchenfalls wird also der Hüftgürtel als solcher nicht verändert, sondern er erhält ein zusätzliches Element in Form des die Sekundärverschlusselemente tragenden Materialabschnitts, der in an sich beliebiger Weise auf das Material des Hüftgürtels aufgebracht werden kann, beispielsweise durch Aufnähen, Aufkleben, Aufsiegeln, Ultraschallschweißen oder dergleichen übliche Fügeverfahren.

Es erweist sich auch als vorteilhaft, wenn sich die Gürtelverschlusselemente und/oder die Sekundärverschlusselemente über im Wesentlichen die gesamte Erstreckung des Hüftgürtels in Längsrichtung, also über die gesamte Breite des Hüftgürtels, erstrecken. Auf diese Weise kann beim Einstellen der Sekundärverschlusselemente ein gleichmäßiger Zug über die gesamte Breite des Gürtels auf den Gürtel ausgeübt werden.

Es ist auch denkbar, dass sich die Gürtelverschlusselemente und/oder die Sekundärverschlusselemente zu ihrem freien Ende hin verjüngen oder verschmälern, um ein Reiben der Ecken dieser Verschlusselemente auf der Haut des Benutzers zu minimieren.

Zum Verkürzen der Umfangslänge des Gürtels unter Verwendung der Sekundärverschlusselemente wird dabei vorzugsweise eine Z-förmige Faltung des Gürtels durch Schließen der Sekundärverschlusselemente bewirkt, die durch den dabei entstehenden Druck des Hüftgürtels gegen die Körperoberfläche des Benutzers und/oder durch Reibung der aneinander anliegenden Abschnitte der Z-förmigen Faltung gehalten wird.

Im Hinblick auf die Schaffung einer guten Passform und eines hohen Tragkomforts erweist es sich als vorteilhaft, wenn der Hüftgürtel mindestens einen elastischen Abschnitt, vorzugsweise in jedem Seitenbereich einen elastischen Abschnitt umfasst, so dass der Hüftgürtel in Hüftumfangsrichtung elastisch dehnbar ist.

Es erweist sich weiter als vorteilhaft, wenn der Hüftgürtel im Bauchbereich und/oder im Rückenbereich im Wesentlichen undehnbar ausgebildet ist.

In einer alternativen Ausführungsform kann es sich jedoch auch als vorteilhaft erweisen, wenn sich ein oder mehrere elastische Abschnitte im Bauchbereich und/oder Rückenbereich befinden, vorzugsweise in Form einer keilförmigen, trapezförmigen oder viereckigen Elastifizierung.

Im Hinblick auf die Bemessung des Hüftgürtels erweist es sich als vorteilhaft, wenn der Hüftgürtel an vorzugsweise jeder Stelle eine Erstreckung in Längsrichtung von wenigstens 5 cm, insbesondere von wenigstens 6 cm, insbesondere von wenigstens 7 cm, insbesondere von wenigstens 8 cm, insbesondere von wenigstens 9 cm und weiter insbesondere von wenigstens 10 cm aufweist. Hierbei ist die Abmessung des Hüftgürtels in Längsrichtung, also die Gürtelbreite quer zu seiner Umfangserstreckung, und zwar außerhalb des vorderen oder hinteren Latzabschnitts gemeint. Die Erstreckung in Längsrichtung ist vorzugsweise höchstens 20 cm. Besonders bevorzugt ist die Erstreckung im Bauchbereich geringer als im Rückenbereich, und der obere Rand des Hüftgürtels ist vorzugsweise leicht konturiert, um einen Bauchausschnitt zu bilden, der die Passform des Produkts verbessert.

Hinsichtlich der Bemessung des Latzabschnitts erweist es sich als vorteilhaft, wenn der Latzabschnitt eine Erstreckung in Längsrichtung über einen schrittzugewandten Umfangsrand des Hüftgürtels von wenigstens 5 cm, insbesondere von wenigstens 6 cm, insbesondere von wenigstens 7 cm, insbesondere von wenigstens 8 cm, insbesondere von wenigstens 9 cm, insbesondere von wenigstens 10 cm, insbesondere von höchstens 20 cm, insbesondere von 10 - 18 cm, insbesondere von 10 - 15 cm aufweist, wobei der vordere und hintere Latzabschnitt die gleiche oder eine unterschiedliche Erstreckung haben können.

Nach einer bevorzugten Ausführungsform der Erfindung ist der betreffende Latzabschnitt einstückig mit dem Bauchbereich und/oder mit dem Rückenbereich des Hüftgürtels ausgebildet. Separat angestückte Latzabschnitte aus dem gleichen Material wie der Hüftgürtel oder aus einem anderen Material als der Hüftgürtel sind aber ebenfalls denkbar.

Der Bauchbereich und/oder Rückenbereich des Hüftgürtels ist vorzugsweise von einem Vliesmaterial oder Vliesverbundmaterial, insbesondere von einem im Wesentlichen undehnbaren Vliesmaterial oder Vliesverbundmaterial, gebildet.

Weiter erweist es sich als besonders vorteilhaft, wenn ein elastisch dehnbarer Seitenbereich des Hüftgürtels mit einem im wesentlichen undehnbaren Bauchbereich und einem im Wesentlichen undehnbaren Rückenbereich unlösbar verbunden ist. Vorzugsweise umfasst jeder Seitenbereich einen elastisch dehnbaren Abschnitt.

Darüber hinaus erweist es sich als vorteilhaft, wenn der vordere und/oder hintere Latzabschnitt farbig ausgebildet ist, wobei die Abschnitte vorzugsweise unterschiedlich farbig sind, um als visuelle Anlegehilfe zu dienen.

Weiterhin ist es vorteilhaft, wenn auf dem vorderen und/oder hinteren Latzabschnitt eine Markierung angebracht ist, die als Positionierhilfe für die Inkontinenzvorlage dient, derart, dass bei korrekt angelegter Inkontinenzvorlage und Fixier- und Tragevorrichtung die Markierung auf dem vorderen und/oder hinteren Latzabschnitt mit einer auf der äußeren Lage der Inkontinenzvorlage sichtbaren Markierung, beispielsweise in Form eines Nässeindikators, in Einklang gebracht ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Fixier- und Tragevorrichtung für eine wegwerfbare absorbierende Inkontinenzvorlage.

In der Zeichnung zeigt:
- Figuren 1 und 2: eine Ausführungsform der erfindungsgemäßen Fixier- und Tragevorrichtung im angelegten Zustand zusammen mit einer wegwerfbaren absorbierenden Inkontinenzvorlage und
- Figur 3: eine schematische Draufsicht auf die Fixier- und Tragevorrichtung im geöffneten und auf eine ebene Unterlage ausgebreiteten Zustand.

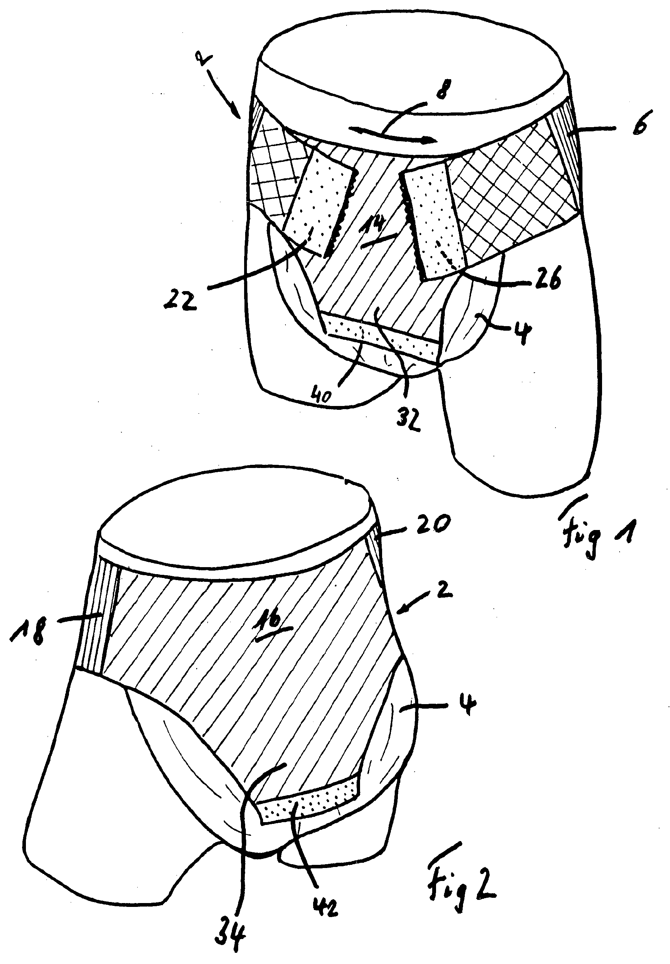

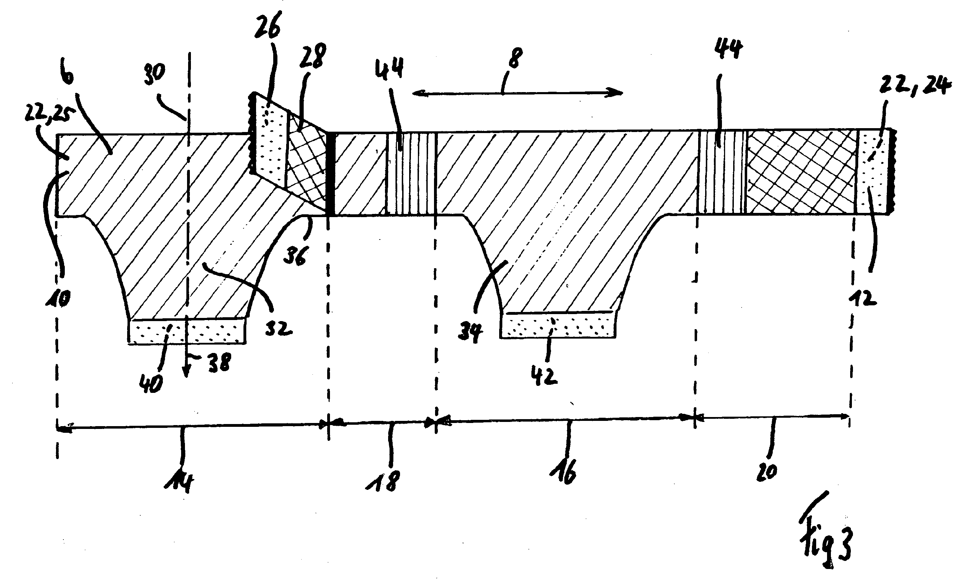

Die Figuren 1 und 2 zeigen perspektivische Ansichten einer insgesamt mit dem Bezugszeichen 2 bezeichneten Fixier- und Tragevorrichtung für eine mit Bezugszeichen 4 angedeutete absorbierende Inkontinenzvorlage, und zwar im an einen Benutzer angelegten Zustand. Die Fixier- und Tragevorrichtung 2 umfasst einen auf sich selbst lösbar schließbaren Hüftgürtel 6, der sich entlang einer Hüftumfangsrichtung 8 erstreckt und zwei freie Endabschnitte 10, 12 (s. Figur 3) aufweist, die zur Bildung der geschlossenen Hüftumfangsform auf sich selbst schließbar sind.

Die Fixier- und Tragevorrichtung 2 bzw. ihr Hüftgürtel 6 umfassen einen vorderen Bauchbereich 14, einen hinteren Rückenbereich 16 sowie einen linken Seitenbereich 18 und einen rechten Seitenbereich 20.

Zum Schließen des Hüftgürtels 6 sind an dessen freien Endabschnitten 10, 12 Gürtelverschlusselemente 22 vorgesehen, die im bevorzugt und beispielhaft dargestellten Fall von mechanisch wirkenden Verschlusselementen in Form eines Haken/Schlaufenverschlusssystems gebildet sind. Dabei ist in den Figuren mit Bezugszeichen 24 die hakenförmige Verschlusskomponente dargestellt, die mit einer schlaufenbildenden Komponente 25 in Form der äußeren Oberfläche des Bauchbereichs 14 lösbar haftend zusammenwirkt. Beispielsweise weist die Außenseite des Bauchbereichs 14 ein Vliesmaterial auf, welches die schlaufenförmige Komponente 25 des Verschlusssystems bildet.

Des Weiteren weist der nur an seinen Endabschnitten 10, 12, also nur an einer Stelle öffenbare Hüftgürtel 6 Sekundärverschlusselemente 26 auf, mittels derer die Umfangslänge des Hüftgürtels 6 einstellbar ist. Die Sekundärverschlusselemente 26 sind seitlich am Bauchbereich 14 bzw. am linken Seitenbereich 18 so vorgesehen, dass sie im angelegten Zustand der Fixier- und Tragevorrichtung (s. Figur 1) denselben optischen Eindruck wie die Gürtelverschlusselemente 22 hinsichtlich Ausbildung und Anordnung vermitteln, obschon ihre Funktion verschieden ist. Mittels der Sekundärverschlusselemente 26 werden nämlich keine öffenbaren und voneinander trennbaren Endabschnitte des Hüftgürtels 6 miteinander verbunden, sondern die Sekundärverschlusselemente 26 dienen lediglich dazu, die Umfangslänge des Hüftgürtels 6 an die Tragesituation anzupassen. Hierfür umfassen die Sekundärverschlusselemente 26 einen auf die Außenseite des Hüftgürtels 6 aufgebrachten Materialabschnitt 28, auf dem oder an dem sie selbst vorgesehen sind. Dieser Materialabschnitt 28 erstreckt sich vorzugsweise über die gesamte Längserstreckung des Gürtels (entlang einer Längsmittelachse 30), also die gesamte Gürtelbreite, um gleichmäßig Zugkräfte in den Hüftgürtel einleiten zu können. Bei den Sekundärverschlusselementen 26 handelt es sich wiederum in bevorzugter Weise um mechanisch wirkende Verschlusselemente, insbesondere um Haken/Schlaufenmaterialien. Im beispielhaft dargestellten und bevorzugten Fall sind die Sekundärverschlusselemente 26 derart ausgebildet, dass an dem Materialabschnitt 28 eine hakenbildende Komponente des Verschlusssystems vorgesehen ist, die mit einer schlaufenbildenden Außenseite des Bauchbereichs 14 zusammenwirkt, so wie dies vorausgehend bei den Gürtelverschlusselementen 22 beschrieben wurde.

Als weitere wesentliche Komponente umfasst der Hüftgürtel 6 ausgehend vom Bauchbereich 14 und vom Rückenbereich 16 je einen symmetrisch zu einer Längsmittelachse 30 ausgebildeten vorderen und hinteren Latzabschnitt 32, 34. Die Latzabschnitte 32, 34 sind im beispielhaft bevorzugt dargestellten Fall einstückig mit dem Hüftgürtel 6 ausgebildet; sie erstrecken sich von einem schrittzugewandten oder unteren Längsrand 36 des Hüftgürtels entlang der Längsmittelachse 30 in Richtung 38 auf den Schrittbereich. Sie können seitlich bogenförmig konturiert ausgebildet sein. An ihrem unteren, schrittzugewandten Endbereich umfassen die Latzabschnitte 32, 34 mechanisch wirkende Verschlusselemente 40, 42, und zwar auf der körperzugewandten Seite. Diese mechanisch wirkenden Verschlusselemente 40, 42 wirken lösbar haftend mit komplementären mechanisch wirkenden Verschlusselementen auf der körperabgewandten Seite der Inkontinenzvorlage 4 zusammen, um die Inkontinenzvorlage 4 lösbar, jedoch verliersicher an der Fixier- und Tragevorrichtung 2 festzulegen. Bevorzugtermaßen handelt es sich bei den Verschlusselementen 40, 42 um die hakenbildende Komponente eines mechanisch wirkenden Verschlusssystems, die mit der schlaufenbildenden Komponenten auf der körperabgewandten Seite der Inkontinenzvorlage 4 zusammenwirkt. Die schlaufenbildende Komponente kann dabei in vorteilhafter Weise von einer Vliesbeschichtung, vorzugsweise der Vlieslage eines Vlies-Folien-Laminates, der Inkontinenzvorlage 4 gebildet sein.

Der Hüftgürtel 6 ist vorzugsweise aus Vlies- oder Vliesverbundmaterialien hergestellt. Im beispielhaft dargestellten jedoch bevorzugten Fall ist der Bauchbereich 14 und der Rückenbereich 16 aus einem im Wesentlichen undehnbaren Vliesmaterial oder Vliesverbundmaterial gebildet, dessen Außenseite, die als schlaufenbildende Komponente der Gürtelverschlusselemente 22 bzw. der Sekundärverschlusselemente 26 dient. Die beiden Seitenbereiche 18, 20 sind hingegen elastisch dehnbar ausgebildet, indem sie einen elastisch dehnbaren Materialabschnitt 44 aufweisen, der bestimmungsgemäß unlösbar mit dem undehnbaren Material des Bauchbereichs 14 und Rückenbereichs 16 verbunden ist.

Das Anlegen der Fixier- und Tragevorrichtung 2 zusammen mit der absorbierenden Inkontinenzvorlage 4 geschieht folgendermaßen: Zunächst wird der Hüftgürtel 6 bei bettlägerigen pflegebedürftigen Patienten durch eine Pflegeperson unter dem Körper des Patienten auf Hüfthöhe hindurchgeführt. Der Hüftgürtel 6 wird dabei so positioniert, dass der Rückenbereich 16 unterhalb des Gesäßes und der Bauchbereich 14 ungefähr vorne mittig zu liegen kommen. Sodann werden die freien Endabschnitte 10, 12 des Hüftgürtels 6 übereinander positioniert und mittels der Gürtelverschlusselemente 22 aufeinander geschlossen. Durch anschließendes Positionieren und Schließen des Materialabschnitts 28 und der Sekundärverschlusselemente 26 wird die Länge und Spannung des Hüftgürtels 6 in Umfangsrichtung 8 optimiert, so dass ein gleichmäßiger Zug auf den Bauchbereich 14 links und rechts ausgeübt wird und der Bauchbereich 14 wie auch der Rückenbereich 16 vorzugsweise symmetrisch in Bezug auf den Patienten und möglichst faltenfrei zu liegen kommen. Hierbei ist auch auf ein angenehmes Traggefühl abzustellen. Sodann wird der vordere und hintere Latzabschnitt 32, 34 mit der zuvor oder erst jetzt im Schrittbereich des Patienten positionierten Inkontinenzvorlage 4 unter Verwendung der mechanisch wirkenden Verschlusselemente 40, 42 lösbar haftend verbunden. Auch hierbei ist darauf zu achten, dass die vorderen und hinteren Latzabschnitte 32, 34 unter Ausübung einer gleichmäßigen und moderaten Zug- bzw. Haltekraft auf die Inkontinenzvorlage 4 fixiert werden, damit die Inkontinenzvorlage 4 in ihrer bestimmungsgemäßen symmetrischen Anordnung im Schrittbereich des Patienten verbleibt.

Hinsichtlich der Reihenfolge der vorausgehend geschilderten Schritte zum Anlegen des Hygieneartikels bzw. Inkontinenzsystems ist die Pflegeperson verhältnismäßig frei. Beispielsweise kann es sich in bestimmten Pflegesituationen als vorteilhaft erweisen, dass zunächst die Inkontinenzvorlage im Schrittbereich des Patienten vorpositioniert wird und erst dann der Hüftgürtel 6 auf sich selbst geschlossen wird. Es kann sich auch als zweckmäßig erweisen, dass die Sekundärverschlusselemente 26 erst nach dem Verbinden der Latzabschnitte 32, 34 mit der Inkontinenzvorlage 4 genutzt werden, um die optimale Spannung in Hüftumfangsrichtung 8 vorzugeben.

## Patentansprüche

1. Fixier- und Tragevorrichtung (2) für eine wegwerfbare absorbierende Inkontinenzvorlage (4), mit einem mittels Gürtelverschlusselementen (22) auf sich selbst lösbar schließbaren und so eine in Hüftumfangsrichtung (8) durchgehende Hüftöffnung bildenden Hüftgürtel (6), an den die Inkontinenzvorlage (4) lösbar fixierbar ist, so dass sie im Schrittbereich des Benutzers getragen und nach Gebrauch wieder vom Hüftgürtel (6) entfernt und verworfen werden kann, wobei der Hüftgürtel (6) einen vorderen Bauchbereich (14), einen hinteren Rückenbereich (16) und einen linken und einen rechten Seitenbereich (18, 20) umfasst, wobei der Hüftgürtel (6) ausgehend von dem Rückenbereich (16) und dem Bauchbereich (14) je einen sich in einer Längsrichtung in Richtung (38) auf den Schrittbereich des Benutzers erstreckenden Latzabschnitt (32, 34) aufweist, der an seiner körperzugewandten Seite vorzugsweise mechanisch wirkende Verschlusselemente (40, 42) aufweist, die mit komplementären vorzugsweise mechanisch wirkenden Verschlusselementen auf der körperabgewandten Seite der Inkontinenzvorlage (4) lösbar haftend zusammenwirken, **dadurch gekennzeichnet, dass** der Hüftgürtel (6) nur in einem Bereich öffenbar und schließbar und die Gürtelverschlusselemente (22) in diesem Bereich an freien Endabschnitten (10, 12) des Hüftgürtels (6) vorgesehen sind und dass Sekundärverschlusselemente (26) vorgesehen sind, mittels derer eine Umfangslänge des Hüftgürtels (6) einstellbar und so zumindest der vordere Latzabschnitt (32) symmetrisch zum Schritt des Benutzers positionierbar ist, und dass die Gürtelverschlusselemente (22) und die Sekundärverschlusselemente (26) beidseits des Bauchbereichs (14) oder in Seitenbereichen (18, 20) des Hüftgürtels (6) vorgesehen sind.

2. Fixier- und Tragevorrichtung nach Anspruch 1, **dadurch gekennzeichnet, dass** der Latzabschnitt (32, 34) sich in Richtung (38) auf den Schrittbereich verjüngt.

3. Fixier- und Tragevorrichtung nach Anspruch 1 oder 2, **dadurch gekennzeichnet, dass** die Verschlusselemente (40, 42) des Latzabschnitts (32, 34) zumindest an einem schrittzugewandten Ende des Latzabschnitts vorgesehen sind.

4. Fixier- und Tragevorrichtung nach einem oder mehreren der vorstehenden Ansprüche, **dadurch gekennzeichnet, dass** die Verschlusselemente (40, 42) des Latzabschnitts (32, 34) auf einem streifenförmigen und quer zur Längsrichtung erstreckten Materialabschnitt vorgesehen sind, der an den Latzabschnitt (32, 34) angefügt ist.

5. Fixier- und Tragevorrichtung nach einem oder mehreren der vorstehenden Ansprüche, **dadurch gekennzeichnet, dass** die Verschlusselemente (40, 42) des Latzabschnitts (32, 34) vor der Ingebrauchnahme auf die körperzugewandte Seite des Latzabschnitts (32, 34) bzw. auf den Hüftgürtel (6) eingeschlagen und in dieser Konfiguration leicht lösbar an der körperzugewandte Seite des Latzabschnitts (32, 34) oder des Hüftgürtels (6) haftend sind.

6. Fixier- und Tragevorrichtung nach einem oder mehreren der vorstehenden Ansprüche, **dadurch gekennzeichnet, dass** eine Komponente der Sekundärverschlusselemente (26) auf einem an den Hüftgürtel angefügten Materialabschnitt (28) vorgesehen ist und die andere Komponente an einer Außenseite des Hüftgürtels (6) vorgesehen ist.

7. Fixier- und Tragevorrichtung nach einem oder mehreren der vorstehenden Ansprüche, **dadurch gekennzeichnet, dass** sich die Gürtelverschlusselemente (22) und/oder die Sekundärverschlusselemente (26) über im Wesentlichen die gesamte Erstreckung des Hüftgürtels (6) in Längsrichtung (30) erstrecken.

8. Fixier- und Tragevorrichtung nach einem oder mehreren der vorstehenden Ansprüche, **dadurch gekennzeichnet, dass** der Hüftgürtel (6) mindestens einen elastischen Abschnitt (44), vorzugsweise in jedem Seitenbereich (18, 20), einen elastischen Abschnitt (44) umfasst, so dass der Hüftgürtel (6) in Hüftumfangsrichtung (8) elastisch dehnbar ist.

9. Fixier- und Tragevorrichtung nach einem oder mehreren der vorstehenden Ansprüche, **dadurch gekennzeichnet, dass** der Hüftgürtel (6) im Bauchbereich (14) und/oder im Rückenbereich (16) im Wesentlichen undehnbar ausgebildet ist.

10. Fixier- und Tragevorrichtung nach einem oder mehreren der vorstehenden Ansprüche, **dadurch gekennzeichnet, dass** der Hüftgürtel (6) an vorzugsweise jeder Stelle eine Erstreckung in Längsrichtung (30) von wenigstens 5 cm, insbesondere von wenigstens 6 cm, insbesondere von wenigstens 7 cm, insbesondere von wenigstens 8 cm, insbesondere von wenigstens 9 cm und weiter insbesondere von wenigstens 10 cm aufweist.

11. Fixier- und Tragevorrichtung nach einem oder mehreren der vorstehenden Ansprüche, **dadurch gekennzeichnet, dass** der Latzabschnitt (32, 34) eine Erstreckung in Längsrichtung (30) über einen schrittzugewandten Umfangsrand des Hüftgürtels von wenigstens 5 cm, insbesondere von wenigstens 6 cm, insbesondere von wenigstens 7 cm, insbesondere von wenigstens 8 cm, insbesondere von wenigstens 9 cm, insbesondere von wenigstens 10 cm, insbesondere von höchstens 20 cm, insbesondere von 10 - 18 cm, insbesondere von 10 - 15 cm aufweist.

12. Fixier- und Tragevorrichtung nach einem oder mehreren der vorstehenden Ansprüche, **dadurch gekennzeichnet, dass** der betreffende Latzabschnitt (32, 34) einstückig mit dem Bauchbereich (14) und/oder mit dem Rückenbereich (16) des Hüftgürtels (6) ausgebildet ist.

13. Fixier- und Tragevorrichtung nach einem oder mehreren der vorstehenden Ansprüche, **dadurch gekennzeichnet, dass** der Bauchbereich (14) und/oder der Rückenbereich (16) des Hüftgürtels (6) von einem Vliesmaterial oder Vliesverbundmaterial gebildet ist.

14. Fixier- und Tragevorrichtung nach einem oder mehreren der vorstehenden Ansprüche, **dadurch gekennzeichnet, dass** ein elastisch dehnbarer Seitenbereich (18, 20) des Hüftgürtels (6) mit einem im wesentlichen undehnbaren Bauchbereich (14) und einem im Wesentlichen undehnbaren Rückenbereich (16) unlösbar verbunden ist.

## Claims

1. Fixing and carrying device (2) for a disposable absorbent incontinence pad (4), having a girdle (6) which is detachably closable on itself by means of girdle closure elements (22) and thus forms a continuous hip opening in the hip circumferential direction (8), the incontinence pad (4) being detachably fixable to said girdle (6) such that it can be worn in the crotch region of the user and can be removed from the girdle (6) again and discarded after use, wherein the girdle (6) comprises a front stomach region (14), a rear back region (16) and a left-hand and a right-hand side region (18, 20), wherein the girdle (6), starting from the back region (16) and the stomach region (14), has in each case one flap section (32, 34) extending in a longitudinal direction in the direction (38) of the crotch region of the user, said flap section (32, 34) having, on its side facing the body, preferably mechanically acting closure elements (40, 42) which interact in a detachably adhesive manner with complementary preferably mechanically acting closure elements on that side of the incontinence pad (4) that faces away from the body, **characterized in that** the girdle (6) is openable and closable only in one region and the girdle closure elements (22) are provided in this region on free end sections (10, 12) of the girdle (6) and **in that** secondary closure elements (26) are provided, by means of which a circumferential length of the girdle (6) is settable and thus at least the front flap section (32) is positionable symmetrically with respect to the crotch of the user, and **in that** the girdle closure elements (22) and the secondary closure elements (26) are provided on both sides of the stomach region (14) or in side regions (18, 20) of the girdle (6).

2. Fixing and carrying device according to Claim 1, **characterized in that** the flap section (32, 34) narrows in the direction (38) of the crotch region.

3. Fixing and carrying device according to Claim 1 or 2, **characterized in that** the closure elements (40, 42) of the flap section (32, 34) are provided at least at an end, facing the crotch, of the flap section.

4. Fixing and carrying device according to one or more of the preceding claims, **characterized in that** the closure elements (40, 42) of the flap section (32, 34) are provided on a strip-like material section extending transversely to the longitudinal direction, said material section being joined to the flap section (32, 34).

5. Fixing and carrying device according to one or more of the preceding claims, **characterized in that** the closure elements (40, 42) of the flap section (32, 34) are folded in, prior to being put into use, on that side of the flap section (32, 34) that faces the body or on the girdle (6), and in this configuration adhere in an easily detachable manner to that side of the flap section (32, 34) or of the girdle (6) that faces the body.

6. Fixing and carrying device according to one or more of the preceding claims, **characterized in that** one component of the secondary closure elements (26) is provided on a material section (28) joined to the girdle and the other component is provided on an outer side of the girdle (6).

7. Fixing and carrying device according to one or more of the preceding claims, **characterized in that** the girdle closure elements (22) and/or the secondary closure elements (26) extend over substantially the entire extent of the girdle (6) in the longitudinal direction (30).

8. Fixing and carrying device according to one or more of the preceding claims, **characterized in that** the girdle (6) comprises at least one elastic section (44), preferably an elastic section (44) in each side region (18, 20), such that the girdle (6) is elastically stretchable in the hip circumferential direction (8).

9. Fixing and carrying device according to one or more of the preceding claims, **characterized in that** the girdle (6) is formed in a substantially non-stretchable manner in the stomach region (14) and/or in the back region (16).

10. Fixing and carrying device according to one or more of the preceding claims, **characterized in that** the girdle (6) has, at preferably any point, an extent in the longitudinal direction (30) of at least 5 cm, in particular of at least 6 cm, in particular of at least 7 cm, in particular of at least 8 cm, in particular of at least 9 cm and more particularly of at least 10 cm.

11. Fixing and carrying device according to one or more of the preceding claims, **characterized in that** the flap section (32, 34) has an extent in the longitudinal direction (30) over a circumferential edge, facing the crotch, of the girdle of at least 5 cm, in particular of at least 6 cm, in particular of at least 7 cm, in particular of at least 8 cm, in particular of at least 9 cm, in particular of at least 10 cm, in particular of at most 20 cm, in particular of 10 - 18 cm, in particular of 10 - 15 cm.

12. Fixing and carrying device according to one or more of the preceding claims, **characterized in that** the relevant flap section (32, 34) is formed in one piece with the stomach region (14) and/or with the back region (16) of the girdle (6).

13. Fixing and carrying device according to one or more of the preceding claims, **characterized in that** the stomach region (14) and/or the back region (16) of the girdle (6) is formed from a nonwoven material or nonwoven composite material.

14. Fixing and carrying device according to one or more of the preceding claims, **characterized in that** an elastically stretchable side region (18, 20) of the girdle (6) is connected non-detachably to a substantially non-stretchable stomach region (14) and a substantially non-stretchable back region (16).

## Revendications

1. Dispositif de support et de fixation (2) pour une protection pour incontinence absorbante jetable (4), comprenant une gaine (6) qui peut se refermer sur elle-même de manière détachable au moyen d'éléments de fermeture de gaine (22) et qui forme ainsi une ouverture continue pour les hanches dans la direction périphérique des hanches (8), sur laquelle gaine peut être fixée de manière détachable la protection pour incontinence (4) de manière à ce qu'elle puisse être portée par l'utilisateur dans la région de la fourche et qu'elle puisse être ensuite enlevée de la gaine (6) et jetée après l'utilisation, la gaine (6) comprenant une région ventrale avant (14), une région dorsale arrière (16) et des régions latérales gauche et droite (18, 20), la gaine (6), partant de la région dorsale (16) et de la région ventrale (14), présentant une partie de volet (32, 34) respective s'étendant dans une direction longitudinale dans la direction (38) de la région de fourche de l'utilisateur, laquelle partie de volet présente sur son côté tourné vers le corps de préférence des éléments de fermeture mécaniques (40, 42) qui coopèrent de manière adhésive et détachable avec des éléments complémentaires de fermeture de préférence mécaniques sur le côté opposé au corps de la protection pour incontinence (4), **caractérisé en ce que** la gaine (6) ne peut être ouverte et fermée que dans une région et les éléments de fermeture de gaine (22) sont prévus dans cette région au niveau de parties d'extrémité libres (10, 12) de la gaine (6) et **en ce que** des éléments de fermeture secondaires (26) sont prévus, au moyen desquels une longueur périphérique de la gaine (6) peut être ajustée et donc au moins la partie de volet avant (32) peut être positionnée symétriquement par rapport à la fourche de l'utilisateur, et **en ce que** les éléments de fermeture de gaine (22) et les éléments de fermeture secondaires (26) sont prévus de chaque côté de la région ventrale (14) ou dans les régions latérales (18, 20) de la gaine (6).

2. Dispositif de support et de fixation selon la revendication 1, **caractérisé en ce que** la partie de volet (32, 34) se rétrécit dans la direction (38) de la région de fourche.

3. Dispositif de support et de fixation selon la revendication 1 ou 2, **caractérisé en ce que** les éléments de fermeture (40, 42) de la partie de volet (32, 34) sont prévus au moins au niveau d'une extrémité de la partie de volet tournée vers la fourche.

4. Dispositif de support et de fixation selon l'une quelconque ou plusieurs des revendications précédentes, **caractérisé en ce que** les éléments de fermeture (40, 42) de la partie de volet (32, 34) sont prévus sur une partie de matériau en forme de bande s'étendant transversalement à la direction longitudinale, laquelle est assemblée à la partie de volet (32, 34).

5. Dispositif de support et de fixation selon l'une quelconque ou plusieurs des revendications précédentes, **caractérisé en ce que** les éléments de fermeture (40, 42) de la partie de volet (32, 34) avant l'utilisation sont repliés sur le côté tourné vers le corps de la partie de volet (32, 34) ou sur la gaine (6) et sont maintenus en adhérence dans cette configuration de manière facilement détachable sur le côté tourné vers le corps de la partie de volet (32, 34) ou de la gaine (6).

6. Dispositif de support et de fixation selon l'une quelconque ou plusieurs des revendications précédentes, **caractérisé en ce qu'**un composant des éléments de fermeture secondaires (26) est prévu sur une partie de matériau (28) assemblée à la gaine et l'autre composant est prévu sur un côté extérieur de la gaine (6).

7. Dispositif de support et de fixation selon l'une quelconque ou plusieurs des revendications précédentes, **caractérisé en ce que** les éléments de fermeture de gaine (22) et/ou les éléments de fermeture secondaires (26) s'étendent dans la direction longitudinale (30) essentiellement sur toute l'étendue de la gaine (6).

8. Dispositif de support et de fixation selon l'une quelconque ou plusieurs des revendications précédentes, **caractérisé en ce que** la gaine (6) comprend au moins une partie élastique (44), de préférence une partie élastique (44) dans chaque région latérale (18, 20), de telle sorte que la gaine (6) puisse être étirée élastiquement dans la direction périphérique des hanches (8).

9. Dispositif de support et de fixation selon l'une quelconque ou plusieurs des revendications précédentes, **caractérisé en ce que** la gaine (6) est réalisée dans la région ventrale (14) et/ou dans la région dorsale (16) de manière essentiellement non étirable.

10. Dispositif de support et de fixation selon l'une quelconque ou plusieurs des revendications précédentes, **caractérisé en ce que** la gaine (6) présente, de préférence en chaque point, une étendue dans la direction longitudinale (30) d'au moins 5 cm, en particulier d'au moins 6 cm, en particulier d'au moins 7 cm, en particulier d'au moins 8 cm, en particulier d'au moins 9 cm et plus particulièrement d'au moins 10 cm.

11. Dispositif de support et de fixation selon l'une quelconque ou plusieurs des revendications précédentes, **caractérisé en ce que** la partie de volet (32, 34) présente une étendue dans la direction longitudinale (30) sur un bord périphérique de la gaine tourné vers la fourche d'au moins 5 cm, en particulier d'au moins 6 cm, en particulier d'au moins 7 cm, en particulier d'au moins 8 cm, en particulier d'au moins 9 cm, en particulier d'au moins 10 cm, en particulier d'au plus 20 cm, en particulier de 10 - 18 cm, en particulier de 10 - 15 cm.

12. Dispositif de support et de fixation selon l'une quelconque ou plusieurs des revendications précédentes, **caractérisé en ce que** la partie de volet concernée (32, 34) est réalisée d'une seule pièce avec la région ventrale (14) et/ou avec la région dorsale (16) de la gaine (6).

13. Dispositif de support et de fixation selon l'une quelconque ou plusieurs des revendications précédentes, **caractérisé en ce que** la région ventrale (14) et/ou la région dorsale (16) de la gaine (6) est formée par un matériau non tissé ou par un matériau composite non tissé.

14. Dispositif de support et de fixation selon l'une quelconque ou plusieurs des revendications précédentes, **caractérisé en ce qu'**une région latérale étirable élastiquement (18, 20) de la gaine (6) est connectée de manière non détachable à une région ventrale (14) essentiellement non étirable et à une région dorsale (16) essentiellement non étirable.
